# EUROPEAN PATENT APPLICATION

(11) **EP 4 006 505 A1**
(43) Date of publication of application: **01.06.2022**
(21) Application number: 20847105.2
(22) Date of filing: 29.07.2020
(51) Int. Cl.: G01H 3/00

(54) **EVALUATION SYSTEM, EVALUATION APPARATUS, EVALUATION METHOD, PROGRAM, AND SOUND INSULATION DEVICE**

(30) Priority: 31.07.2019 JP 2019141547
(71) Applicant: Nitto Denko Corporation, Ibaraki-shi, Osaka 567-8680 (JP)
(72) Inventor: HATANAKA, Takezo, Ibaraki-shi, Osaka 567-8680 (JP)
(74) Representative: Hoffmann Eitle
(86) International application number: PCT/JP2020/029008
(87) International publication number: WO 2021/020430

(57) **Abstract**

An assessment system includes a sound data acquiring unit configured to acquire sound data collected by a sound insulation device, the sound insulation device being fitted into a wearer's ears; and an assessment unit configured to assess noise to which the wearer is exposed, based on the sound data acquired by the sound data acquiring unit and time information associated with the sound data.

## Description

### TECHNICAL FIELD

The present invention relates to an assessment system, an assessment device, an assessment method, a program, and a sound insulation device.

### BACKGROUND ART

In the related art, measures have been taken in noisy environments at construction sites and factories, such as ensuring that workers wear earplugs, and measuring noise using noise measuring instruments, in consideration of workers and surrounding environments of the workers.

For example, Patent Document 1 discloses a construction management system in which a portable terminal, a server device, and a terminal device communicate with each other via a network, wherein the system displays noise measurement information by a noise measuring device on the portable terminal.

Patent Document 2 discloses a noise monitoring system for noise generated during construction work in which a noise measuring unit for measuring a level of noise generated in a construction area is installed to calculate and display the level of the arrived noise at any monitoring position.

Patent Document 3 discloses a technique for measuring sound pressure levels inside and outside an individual's external auditory canals by means of a probe microphone and a reference microphone included in an intraocular device inserted into the individual's external auditory canals.

### RELATED ART DOCUMENTS

### [Patent Documents]

[Patent Document 1] Japanese Unexamined Patent Application Publication No. 2018-163423
[Patent Document 2] Japanese Patent No. 6305254
[Patent Document 3] Japanese Translation of PCT International Application Publication No. JP-T-2004-524070

### SUMMARY OF THE INVENTION

### [PROBLEM TO BE SOLVED BY THE INVENTION]

In the related art techniques, however, the original sound insulating performance may not be achieved unless a worker wears the earplugs correctly. In addition, the related art techniques have problems in that installation of a number of noise measuring instruments in noise generating locations is required. That is, the related art techniques cannot appropriately assess noise, to which the wearer of the earplugs is actually exposed, including time factors, based on noise data of the noise to which the wearer is actually exposed.

Accordingly, one aspect of the present invention is intended to more appropriately perform assessments associated with noise, to which wearers of earplugs are actually exposed, including time factors, based on sound data associated with the noise to which the wearers are actually exposed.

### [MEANS FOR SOLVING THE PROBLEM]

In one aspect according to the present invention, an assessment system according to an embodiment of the present invention includes
a sound data acquiring unit configured to acquire sound data collected by a sound insulation device, the sound insulation device being fitted into a wearer's ears; and
an assessment unit configured to assess noise to which the wearer is exposed, based on the sound data acquired by the sound data acquiring unit and time information associated with the sound data.

### [ADVANTAGEOUS EFFECT OF THE PRESENT INVENTION]

In one aspect according to the present invention, it is possible to more appropriately perform an assessment associated with noise, to which wearers of earplugs are actually exposed, including time factors, based on sound data of noise to which the wearers are actually exposed.

### [BRIEF DESCRIPTION OF THE DRAWINGS]

FIG. 1 is a diagram illustrating a system configuration of an assessment system according to a first embodiment of the present invention;
FIG. 2 is a diagram illustrating a configuration of a sound insulation device according to the first embodiment of the present invention;
FIG. 3A is a diagram illustrating an example of a hardware configuration of a sound insulation device;
FIG. 3B is a diagram illustrating an example of a hardware configuration of a server and a terminal;
FIG. 4 is a block diagram illustrating a functional configuration of a server according to a first embodiment of the present invention;
FIG. 5 is a flowchart illustrating an example (first example) of a procedure for assessing a sound insulation effect by the server according to the first embodiment of the present invention;
FIG. 6 is a flowchart illustrating an example (second example) of a procedure for assessing a sound insulation effect by the server according to the first embodiment of the present invention;
FIG. 7 is a diagram illustrating an example of a display screen (first example) displayed on a terminal device according to the first embodiment of the present invention;
FIG. 8 is a flowchart illustrating an example of a process for assessing a risk by the server according to the first embodiment of the present invention;
FIG. 9 is a diagram illustrating an example of assessment result information output by an assessment result output unit according to the first embodiment of the present invention;
FIG. 10 is a diagram illustrating an example (second example) of assessment result information output by the assessment result output unit according to the first embodiment of the present invention;
FIG. 11 is a diagram illustrating an example (second example) of a display screen displayed on the terminal device according to the first embodiment of the invention;
FIG. 12 is a diagram illustrating a system configuration of an assessment system according to a second embodiment of the invention; and
FIG. 13 is a diagram illustrating a system configuration of an assessment system according to a third embodiment of the invention.

### EMBODIMENTS FOR CARRYING OUT THE INVENTION

In the following, embodiments according to the present invention will be described in detail with reference to the accompanying drawings.

### [First Embodiment]

### (System configuration of assessment system 10)

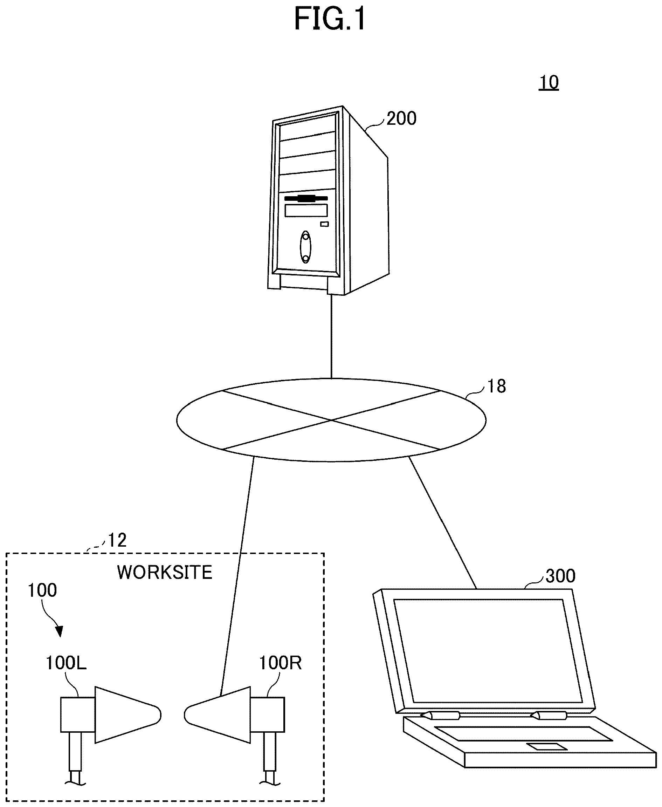

FIG. 1 is a diagram illustrating a system configuration of an assessment system 10 according to a first embodiment of the present invention. The assessment system 10 illustrated in FIG. 1 is a system capable of performing various assessments including time factors associated with noise to which workers are actually exposed, based on sound data of noise to which each of a plurality of workers (an example of a "wearer") is exposed at a worksite 12.

As illustrated in FIG. 1, the assessment system 10 includes a sound insulation device 100, a server 200, and a terminal device 300. The sound insulation device 100, the server 200, and the terminal device 300 are all connectable to a communication network 18 and are capable of communicating with other devices via the communication network 18. As the communication network 18, for example, the Internet, a LAN (local area network), a VPN (virtual private network), or the like is used.

### <Sound insulation device 100>

To prevent noise-induced hearing loss, the sound insulation device 100 is a device capable of measuring sound pressure levels outside and inside the external auditory canals while protecting the ears from noise. The sound insulation device 100 includes a fitting portion 100L and a fitting portion 100R. The fitting portion 100L is fitted into the worker's left ear. The fitting portion 100R is fitted into the worker's right ear. The fitting portions 100L and 100R function as earplugs. The fitting portions 100L and 100R can be fitted into the worker's ears to shield the worker's external auditory canals from noise to which the worker is exposed.

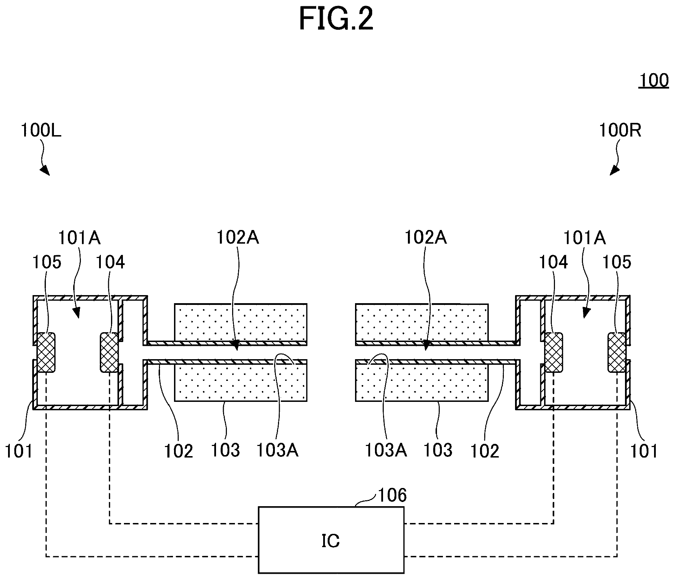

Each of the fitting portions 100L and 100R has a built-in first microphone 104 and a built-in second microphone 105 (see FIG. 2). This enables the sound insulation device 100 to continuously acquire sounds inside and outside the external auditory canal for each of the worker's left and right ears.

The sound insulation device 100 may store sound data acquired by the first microphone 104 and the second microphone 105 in memory or transmit the sound data to the server 200 and the terminal device 300 via the communication network 18. Although FIG. 1 illustrates one sound insulation device 100 for convenience, in practice, the assessment system 10 includes a plurality of sound insulation devices 100 to be worn by the plurality of workers.

Further, the sound insulation device 100 is an example of an "assessment device". For each of the fitting portions 100L and 100R, the following assessment can be performed, based on the sound pressure level inside the external auditory canals of a worker acquired by the first microphone 104 and the sound pressure level outside the external auditory canals of the worker acquired by the second microphone 105.

- Assessment of sound insulation effect: Assessment of sound insulation effect by sound insulation device 100

### <Server 200>

A server 200 is another example of an "assessment device" and is a device capable of storing sound data collected from each of a plurality of sound insulation devices 100 and capable of performing various noise-related assessments for each of a plurality of workers, based on the accumulated sound data. For example, in this embodiment, the server 200 can perform the following two assessments.

- Assessment of sound insulation effect: Assessment of sound insulation effect by sound insulation device 100
- Risk assessment: Noise-induced risk assessment with respect to workers at a worksite 12

The server 200 also has the following two functions.

- Abnormal sound detection function: Function to detect abnormal sound from sound data
- Utterance signal extraction function: Function to extract utterance signals from sound data

### <Terminal device 300>

A terminal device 300 is used by a manager at a worksite 12. For example, the terminal device 300 performs various settings for the server 200 and displays various assessment results obtained from the server 200. For example, a PC (Personal Computer), a tablet terminal, a smartphone, or the like may be used as the terminal device 300. Further, the terminal device 300 is another example of an "assessment device", and the following assessment can be performed, based on the sound data acquired from the sound insulation device 100.

- Assessment of sound insulation effect: Assessment of sound insulation effect by sound insulation device 100

Although FIG. 1 illustrates one server 200, the assessment system 10 may include a plurality of servers 200. Although FIG. 1 illustrates one terminal device 300, the assessment system 10 may include a plurality of terminal devices 300.

### (Example of configuration of sound insulation device 100)

FIG. 2 is a diagram illustrating an example of a configuration of the sound insulation device 100 according to the first embodiment of the present invention. In the example illustrated in FIG. 2, in the sound insulation device 100, each of the fitting portions 100L and 100R includes a housing 101, a tube 102, a sound insulation member 103, a first microphone 104, and a second microphone 105. The sound insulation device 100 also includes an IC 106.

The housing 101 is a container-like member. An internal space 101A of the housing 101 accommodates the first microphone 104 and the second microphone 105. The housing 101 is made, for example, of a relatively rigid material (e.g., resin) .

The tube 102 is a cylindrical member extending from the housing 101 to the worker's external auditory canal. An internal space 102A of the tube 102 is connected to the internal space 101A of the housing 101. This enables the tube 102 to direct the sound inside the worker's external auditory canal to the internal space 101A of the housing 101. The tube 102 is made, for example, of a resilient material (e.g., silicone, etc.).

The sound insulation member 103 is attached around the tube 102 by passing the tube 102 through a through-hole 103A formed in the sound insulation member 103. The sound insulation member 103 shields noise from entering the worker's external auditory canal from outside the external auditory canal. The sound insulation member 103 is made of a material having sound insulating properties (e.g., sponge, etc.).

The first microphone 104 is disposed on the tube 102 side, i.e., on the worker's external auditory canal side, in the internal space 101A of the housing 101. The first microphone 104 acquires sound inside the worker's external auditory canal that is propagated through the internal space 102A of the tube 102.

The second microphone 105 is disposed opposite to the first microphone 104 connecting to the tube 102, i.e., disposed on the external environment side, in the internal space 101A of the housing 101. The second microphone 105 acquires sound outside the worker's external auditory canal, i.e., the noise to which the worker is exposed.

It should be noted that the first microphone 104 and the second microphone 105 are provided with an acoustic circuit which is not illustrated. The acoustic circuit is configured to include an A/D (Analog to Digital) converter or the like. This enables the first microphone 104 and the second microphone 105 to convert the acquired sound (analog signal) into a digital signal by an acoustic circuit, and output the digital signal as sound data.

The IC 106 performs various controls for sound insulation device 100. For example, the IC 106 may assess the sound insulation effect of the sound insulation device 100, based on the sound pressure level of sound inside the worker's external auditory canal acquired by the first microphone 104 and the sound pressure level of noise acquired by the second microphone 105 for each of the fitting portions 100L and 100R.

The sound insulation device 100 configured in this manner functions as earplugs to block noise when a sound insulation member 103 of each of the fitting portions 100L and 100R is inserted into the external auditory canal of a corresponding ear of the worker.

In each of the fitting portions 100L and 100R of the sound insulation device 100, the first microphone 104 is capable of acquiring sound inside the external auditory canal of the worker, and the second microphone 105 is capable of acquiring noise to which the worker is exposed.

Then, the sound insulation device 100 may be capable of assessing the sound insulation effect of the sound insulation device 100 through the IC 106, based on the sound pressure level of the sound inside the external auditory canal of the worker acquired by the first microphone 104 and the sound pressure level of the noise acquired by the second microphone 105, for each of the fitting portions 100L and 100R.

The sound insulation device 100 is capable of outputting sound data (the external auditory canal internal sound data and the external auditory canal external sound data for each of the fitting portions 100L and 100R) stored in the sound insulation device 100 to an external device (e.g., the server 200 and the terminal device 300) through wireless communication.

It should be noted that the sound insulation device 100 may not necessarily include the second microphone 105 (i.e., the sound insulation device 100 may be configured to acquire the external auditory canal internal sound data alone). Or, the sound insulation device 100 may be provided with the second microphone 105 external to the housing 101.

### (Hardware configuration of each device included in the assessment system 10)

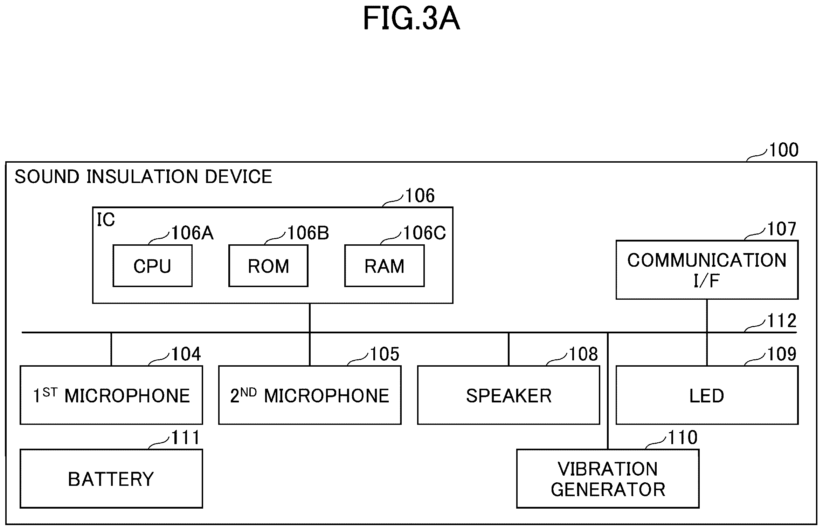

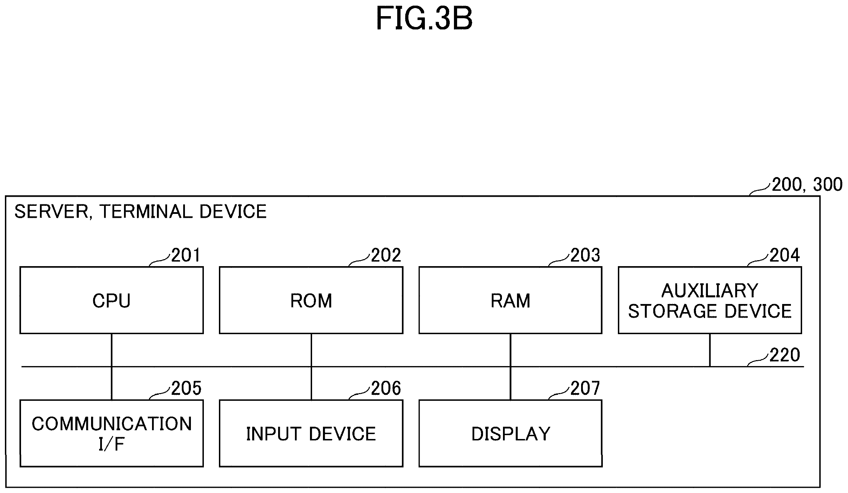

FIGS. 3A and 3B are diagrams illustrating a hardware configuration of each device included in the assessment system 10 according to the first embodiment of the present invention. FIG. 3A is a diagram illustrating an example of a hardware configuration of the sound insulation device 100. FIG. 3B is a diagram illustrating an example of a hardware configuration of the server 200 and the terminal device 300.

### <Sound insulation device 100>

As illustrated in FIG. 3A, the sound insulation device 100 includes a first microphone 104, a second microphone 105, an IC 106, a communication interface 107, a speaker 108, a light emitting diode (LED) 109, a vibration generator 110, and a battery 111. In the sound insulation device 100, each of the hardware except the battery 111 is interconnected via a bus 112.

Note that as illustrated in FIG. 2, in the sound insulation device 100, the first microphone 104 and the second microphone 105 are disposed in each of the fitting portions 100L and 100R. However, the sound insulation device 100 is not limited to this example. In the sound insulation device 100, at least one of the IC 106, the communication I/F 107, the speaker 108, the LED 109, the vibration generator 110, and the battery 111 is shared by the fitting portions 100L and 100R. However, the configuration of the sound insulation device 100 is not limited to this example; and at least one of the IC 106, the communication I/F 107, the speaker 108, the LED 109, the vibration generator 110, and the battery 111 may be disposed in each of the fitting portions 100L and 100R. Further, at least one of the speaker 108, the LED 109, and the vibration generator 110 may be disposed as a notifying unit configured to notify a worker of information.

In each of the fitting portions 100L and 100R, the first microphone 104 acquires sound inside the worker's external auditory canal. In each of the fitting portions 100L and 100R, the second microphone 105 acquires sound outside the worker's external auditory canal.

The IC 106 includes a CPU (Central Processing Unit) 106A, a ROM (Read-only Memory) 106B, and a RAM (Random Access Memory) 106C. The CPU 106A performs various controls of the sound insulation device 100 by executing various programs. The ROM 106B is a non-volatile memory, and stores various programs and various data used by the CPU 106A. The RAM 106C is a main storage device such as DRAM (Dynamic Random Access Memory) and SRAM (Static Random Access Memory). For example, the RAM 106C is used by the CPU 106A as a temporary evacuation area for various data.

The communication I/F 107 is connected to the communication network 18 through wired or wireless communication. The communication I/F 107 communicates with other devices through the communication network 18. For example, the communication I/F 107 may transmit, via the communication network 18, to the server 200 and the terminal device 300, the worker's external auditory canal internal sound data acquired by the first microphone 104 representing a sound inside the worker's external auditory canal and the worker's external auditory canal external sound data acquired by the second microphone 105 representing a sound outside the worker's external auditory canal. As the communication I/F 107, for example, a wireless communication system such as Wi-Fi (registered trademark), Bluetooth (registered trademark), or NFC (Near Field Communication) may be used.

The speaker 108 provides various notifications to a worker by emitting various sounds controlled from the IC 106. The LED 109 provides various notifications to a worker by emitting various kinds of light controlled from the IC 106. The vibration generator 110 provides various notifications to a worker by generating vibrations controlled from the IC 106.

The battery 111 provides power to each of units of sound insulation device 100. Examples of the battery 111 include a variety of rechargeable secondary batteries (e.g., lithium ion secondary batteries, lithium polymer secondary batteries, nickel hydrogen secondary batteries, etc.).

### <Server 200 and terminal device 300>

As illustrated in FIG. 3B, each of the server 200 and the terminal device 300 include a CPU 201, a ROM 202, a RAM 203, an auxiliary storage device 204, a communication I/F 205, an input device 206, and a display 207. In the server 200 and the terminal device 300, respective hardware units 201 to 207 are interconnected via a bus 220.

The CPU 201 performs various controls of the server 200 and the terminal device 300 by executing various programs. The ROM 202 is a non-volatile memory and stores various programs and data used by the CPU 201. The RAM 203 is a main storage device such as a DRAM or a SRAM. For example, the RAM 203 is used by the CPU 201 as a temporary evacuation area for various data.

The auxiliary storage device 204 is a non-volatile storage device. For example, the auxiliary storage device 204 stores various programs and data used by CPU 201. Examples of the auxiliary storage device 204 include an HDD (hard disk drive) and an SSD (solid state drive).

The communication I/F 205 is connected to the communication network 18 by wired or wireless communication. The communication I/F 205 communicates with other devices through the communication network 18.

The input device 206 is used by a worker to perform various input operations. Examples of the input device 206 includes, for example, a mouse, a button, a keyboard, a touch panel, or the like. The display 207 displays various display screens and the like. Examples of the display 207 include a liquid crystal display, an organic EL (Electro Luminescence) display, and the like.

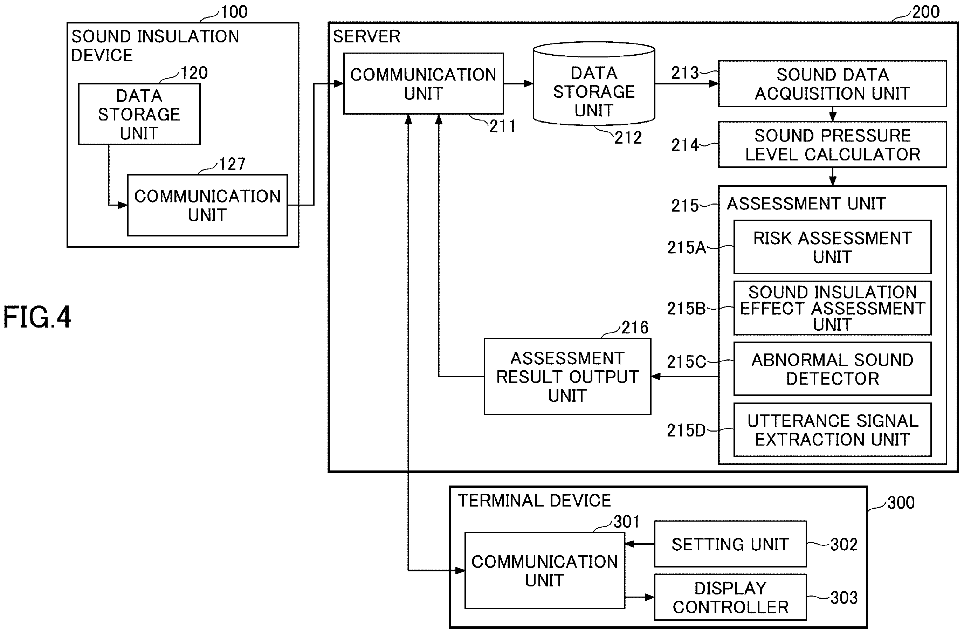

FIG. 4 is a diagram illustrating a functional configuration of each device included in the assessment system 10 according to the first embodiment of the present invention.

### <Functions of sound insulation device 100>

As illustrated in FIG. 4, the sound insulation device 100 includes a data storage unit 120 and a communication unit 127.

The data storage unit 120 stores, for each of the fitting portions 100L and 100R, data acquired by the first microphone 104 and data acquired by the second microphone 105. Since the first microphone 104 and the second microphone 105 of each of the fitting portions 100L and 100R sequentially acquires external auditory canal internal sound data and external auditory canal external sound data, the data storage unit 120 accumulates the external auditory canal internal sound data and the external auditory canal external sound data, every time each of the fitting portions 100L and 100R acquires the external auditory canal internal sound data and the external auditory canal external sound data.

The data storage unit 120 may store each sound data in association with position information representing a current position of the sound insulation device 100 and time information representing the current time. For example, when the sound insulation device 100 includes a GPS (Global Positioning System) (not illustrated), the data storage unit 120 can acquire position information representing the current position of the sound insulation device 100 and time information representing the current time from the GPS. For example, when the position information representing the current position of the sound insulation device 100 (e.g., the work position name or the work area name of the worksite 12) is set to be inside or outside of the sound insulation device 100 (e.g., the server 200, etc.), the data storage unit 120 may acquire the position information from inside or outside of the sound insulation device 100. For example, the data storage unit 120 may acquire time information representing the current time from the system clock (not illustrated) built into the sound insulation device 100.

The association between the sound data and the time information may be made by any device and at any timing. For example, the sound insulation device 100 may associate the sound data with the time information at the timing of storing the sound data in the data storage unit 120. Further, the sound data and time information may, for example, be transmitted from the sound insulation device 100 to the server 200, and the server 200 may associate the sound data received from the sound insulation device 100 with the time information received from the sound insulation device 100. Or, the sound data may be transmitted from the sound insulation device 100 to the server 200, and the server 200 may associate the sound data received from the sound insulation device 100 with time information acquired from the system clock of the server 200. In addition, the time information associated with the sound data may be time integration (elapsed time or cumulative time) instead of the current time.

The communication unit 127 transmits data to and receives data from an external device by communicating with the external device. For example, the communication unit 127 transmits, to the server 200 through the communication network 18, each sound data (i.e., the external auditory canal internal sound data and the external auditory canal external sound data for each of the fitting portions 100L and 100R) stored in the data storage unit 120 together with information associated with each sound data (the position information, the time information, the device ID, etc. of the sound insulation device 100), at a predetermined time or at any time (e.g., every predetermined period such as every hour, every day, etc., at a time when a scheduled time arrives, at a time when a request for transmission is received from the server 200, at a time when a communication connection with the server 200 is made, at a time when a predetermined transmission operation is performed for the sound insulation device 100, etc.).

In the sound insulation device 100, for example, the data storage unit 120 is implemented by the RAM 106C (see FIG. 3A) included in the sound insulation device 100. For example, the communication unit 127 is implemented by the communication I/F 107 (see FIG. 3A) included in the sound insulation device 100.

### <Functions of server 200>

FIG. 4 is a block diagram illustrating a functional configuration of the server 200 according to the first embodiment of the present invention. As illustrated in FIG. 4, the server 200 includes a communication unit 211, a data storage unit 212, a sound data acquisition unit 213, a sound pressure level calculator 214, an assessment unit 215, and an assessment result output unit 216.

The communication unit 211 transmits data to and receives data from the external device by communicating with the external device. For example, the communication unit 211 receives sound data (i.e., external auditory canal internal sound data and external auditory canal external sound data) of each of the plurality of sound insulation devices 100, which are transmitted from each of the plurality of sound insulation devices 100.

The data storage unit 212 stores various types of data. For example, the data storage unit 212 stores the sound data (the external auditory canal internal sound data and the external auditory canal external sound data) of each of the plurality of sound insulation devices 100, which are received by the communication unit 211. That is, the data storage unit 212 stores a plurality of sound data sets collected from each of the plurality of sound insulation devices 100.

The sound data acquisition unit 213 acquires, from the data storage unit 212, sound data (e.g., external auditory canal internal sound data and external auditory canal external sound data) of the sound insulation device 100 (e.g., any one of the sound insulation devices 100, any two or more of the sound insulation devices 100, all the sound insulation devices 100, etc.) to be assessed within an assessment period (e.g., one hour, one day, etc.). The manager of the worksite 12 may set, through the terminal device 300, the sound insulation device 100 to be assessed, the assessment period, and the schedule for executing the assessment, as conditions for acquiring the sound data by the sound data acquisition unit 213.

The sound pressure level calculator 214 calculates a sound pressure level of the sound data acquired by the sound data acquisition unit 213. For example, the sound pressure level calculator 214 calculates the sound pressure level by setting the unit of sound data acquired by the sound data acquisition unit 213 to [decibel (dB)] at every predetermined unit time (e.g., every 1 second). The sound pressure level calculator 214 may be disposed in the sound insulation device 100, and the sound pressure level data may be transmitted to the server 200 by the communication unit 127. In this case, the sound data acquisition unit 213 and the sound pressure level calculator 214 of the server 200 are not required, and the assessment unit 215 can acquire the sound pressure level directly from the data storage unit 212.

The assessment unit 215 assesses noise to which a worker is exposed, based on sound data (and sound pressure level calculated by the sound pressure level calculator 214) acquired by the sound data acquisition unit 213 and time information associated with the sound data. For example, in this embodiment, the assessment unit 215 includes a risk assessment unit 215A, a sound insulation effect assessment unit 215B, an abnormal sound detector 215C, and an utterance signal extraction unit 215D.

The assessment unit 215A assesses a noise-induced risk level with respect to a worker at the worksite 12 based on the sound pressure level calculated by the sound pressure level calculator 214 and time information associated with the sound pressure level.

For example, based on the sound pressure level calculated by the sound pressure level calculator 214 and the time information associated with the sound pressure level, the risk assessment unit 215A assesses a risk level of the sound insulation device 100 (i.e., a worker) to be assessed for every predetermined time unit (e.g., every second, every minute, and every hour) of noise exposure. For example, when the sound pressure level in a predetermined time unit calculated by the sound pressure level calculator 214 is greater than a predetermined threshold value, the risk assessment unit 215A assesses the risk level of the worker in the predetermined time unit as "risk: high". For example, when a sound pressure level in a predetermined time unit calculated by the sound pressure level calculator 214 is lower than a predetermined threshold value, the risk assessment unit 215A assesses the risk level of the worker in a predetermined time unit as "risk level: low".

Further, the risk assessment unit 215A assesses a risk level due to a noise exposure cumulative time of the sound insulation device 100 (i.e., a worker) to be assessed, based on a sound pressure level calculated by the sound pressure level calculator 214 and time information associated with the sound pressure level. For example, based on the sound pressure level calculated by the sound pressure level calculator 214, the risk assessment unit 215A specifies a work start time and a work end time of one or more workers to be assessed. For example, based on the sound pressure level calculated by the sound pressure level calculator 214, the risk assessment unit 215A specifies, as the work start time of the worker, a timing at which the sound pressure level is switched from a period in which the sound pressure level is lower than a predetermined threshold value to a period in which the sound pressure level is greater than the predetermined threshold value. Also, for example, based on the sound pressure level calculated by the sound pressure level calculator 214, the risk assessment unit 215A specifies, as a work end time of a worker, a timing at which the sound pressure level is switched from a period in which the sound pressure level is greater than the predetermined threshold value to a period in which the sound pressure level is lower than the predetermined threshold value. Then, the risk assessment unit 215A calculates a cumulative time of the period in which the sound pressure level is greater than the predetermined threshold value between the specified work start time and the specified work end time, based on the sound pressure level calculated by the sound pressure level calculator 214. In addition, the risk assessment unit 215A assesses a risk level due to the worker's work time, based on the calculated cumulative time. For example, the risk assessment unit 215A assesses the risk level due to the worker's work time such that the longer the calculated cumulative time, the higher the risk level. For example, when the calculated cumulative time is longer than the predetermined time threshold value, the risk assessment unit 215A assesses the risk level due to the worker's work time as "risk: high". For example, when the calculated cumulative time is shorter than the predetermined time threshold value, the risk assessment unit 215A assesses the risk level due to the worker's work time as "risk: low".

However, the risk assessment by the risk assessment unit 215A is not limited to the two levels, but may be three levels or more (e.g., "high", "medium", or "low") by setting multiple thresholds. For example, the risk assessment unit 215A may represent the assessment of the risk level as a numerical value.

The sound data acquired by the sound data acquisition unit 213 is associated with the device ID, the position information, and the time information. Thus, the risk assessment unit 215A can assess the risk level of the worker, the device, the measurement position, and the measurement time, which are substantially associated with the sound data, by assessing the risk level based on the sound data.

Further, the risk assessment unit 215A may assess the noise-induced risk level (the risk level at every predetermined time and the risk level due to noise exposure cumulative time), based on the sound pressure level of the external auditory canal internal sound data alone, the sound pressure level of the external auditory canal external sound data alone, or at least one of the sound pressure level of the external auditory canal internal sound data or the sound pressure level of the external auditory canal external sound data.

The sound insulation effect assessment unit 215B assesses a sound insulation effect of each of the plurality of sound insulation devices 100, based on the sound data of each of the plurality of sound insulation devices 100. Specifically, the sound insulation effect assessment unit 215B assesses a sound insulation effect of one or more sound insulation devices 100 to be assessed for each of the fitting portions 100L and 100R, based on the difference value between a sound pressure level of the external auditory canal internal sound data calculated by the sound pressure level calculator 214 and a sound pressure level of the external auditory canal external sound data calculated by the sound pressure level calculator 214. For example, when the difference value between the sound pressure level of the external auditory canal internal sound data and the sound pressure level of the external auditory canal external sound data is less than a predetermined threshold value, the sound insulation effect assessment unit 215B determines this result as a "poor sound insulation effect". Conversely, when the difference value between the sound pressure level of the external auditory canal internal sound data and the sound pressure level of the external auditory canal external sound data is equal to or greater than the predetermined threshold value, the sound insulation effect assessment unit 215B determines this result as a "normal sound insulation effect". This is because when the sound insulation effect of each of the fitting portions 100L and 100R is normal, the difference value between the sound pressure level of the external auditory canal internal sound data and the sound pressure level of the external auditory canal external sound data becomes equal to or greater than the predetermined threshold value. Accordingly, the predetermined threshold is set to a suitable value that has been previously determined by a test or the like.

The sound insulation effect assessment unit 215B may determine a time at which the "poor sound insulation effect" is determined or a continuation period during which the "poor sound insulation effect" has been determined, based on the time information associated with the sound data. When assessment result information including the above time and the continuation period is output to the terminal device 300, a manager of the worksite 12 can take various actions (e.g., guidance to workers, determination of maintenance of the necessity of maintenance or replacing parts of the sound insulation device 100, etc.) according to the time at which the "poor sound insulation effect" specified from the assessment result information is determined or the continuation period in which the "poor sound insulation effect" has been determined.

The abnormal sound detector 215C detects the abnormal sound at the worksite 12, based on the sound data acquired by the sound data acquisition unit 213. For example, the abnormal sound detector 215C can detect that an abnormal sound (e.g., an abnormality in a working machine, an abnormality in processing, etc.) has been generated at the worksite 12 when the sound pressure level of the external auditory canal external sound data calculated by the sound pressure level calculator 214 is greater than a predetermined threshold value. Note that the abnormal sound detector 215C may detect the abnormal sound by assessing the sound pressure pattern calculated from the sound data rather than the sound pressure level calculated from the sound data.

The abnormal sound detector 215C can specify a location of the abnormal sound, the time at which the abnormal sound is generated, and the continuation period of the abnormal sound, based on the position information and the time information associated with the external auditory canal external sound data. For example, the assessment result output unit 216 outputs the assessment result information including the detection result of the abnormal sound by the abnormal sound detector 215C (the location at which the abnormal sound is generated, the time at which the abnormal sound is generated, the continuation period of the abnormal sound, etc.) to the terminal device 300. This enables the manager of the worksite 12 to take various measures against the generation of the abnormal sound (e.g., determination of the necessity of maintenance or replacing parts of a working machine, instruction to stop the working machine where the abnormal sound is generated, etc.), based on the assessment result information. The abnormal sound detector 215C may not only detect the generation of the abnormal sound, but may also identify a working machine at the source of the abnormal sound, a type of abnormality, or the like by performing a known signal analysis process with respect to voice data.

The utterance signal extraction unit 215D extracts utterance signals from the sound data acquired by the sound data acquisition unit 213. An utterance signal is a voice signal representing speech uttered by a worker, from among the sound data acquired by the sound insulation device 100. For example, due to an utterance of a worker, a voice of the worker may be mixed into the first microphone 104 and the second microphone 105 of the sound insulation device 100, and the external noise may not be accurately assessed as a result. Therefore, the utterance signal extraction unit 215D extracts such utterance signals from sound data acquired by the sound data acquisition unit 213. For example, the utterance signal extraction unit 215D extracts utterance signals from the sound data by performing a known signal analysis process on the sound data acquired by the sound data acquisition unit 213. For example, the utterance signal extraction unit 215D may include the start time and the end time of the utterance signal in the utterance signal extraction result, based on time information associated with the sound data. Then, for example, the utterance signal extraction unit 215D can notify the risk assessment unit 215A, the sound insulation effect assessment unit 215B, and the abnormal sound detector 215C of the extraction result of the utterance signals. For example, the risk assessment unit 215A, the sound insulation effect assessment unit 215B, and the abnormal sound detector 215C may exclude a section including the utterance signals notified from the utterance signal extraction unit 215D, from among the sound data acquired by the sound data acquisition unit 213 that are subject to various assessment processes. Accordingly, the risk assessment unit 215A, the sound insulation effect assessment unit 215B, and the abnormal sound detector 215C can perform various assessment processes based on the voice data (i.e., the voice data including noise alone) not including the voice of the worker, thereby improving the accuracy of various assessment processes.

The assessment result output unit 216 outputs the assessment result information including the assessment result obtained by the assessment unit 215. For example, the assessment result output unit 216 outputs the assessment result information including the assessment result by the assessment unit 215 to the terminal device 300, through the communication unit 211 and the communication network 18. Thus, the terminal device 300 displays the assessment result obtained by the assessment unit 215 on the display 207, which enables the manager of the worksite 12 to understand the assessment result. For example, the assessment result output unit 216 outputs the assessment result information including the assessment result by the risk assessment unit 215A (i.e., the noise-induced risk level at every predetermined time unit and the noise-induced risk level exposure cumulative time, with respect to one or more sound insulation devices 100 to be assessed) to the terminal device 300. For example, the assessment result output unit 216 outputs the assessment result information including the assessment result (i.e., the sound insulation effect on one or more sound insulation devices 100 to be assessed) by the sound insulation effect assessment unit 215B to the terminal device 300. For example, the assessment result output unit 216 outputs the assessment result information including the assessment result (i.e., the detection result of the abnormal sound at the worksite 12) by the abnormal sound detector 215C to the terminal device 300.

The assessment result information output from the assessment result output unit 216 does not include the sound pressure level or the like as the assessment result, but includes the assessment result of a display format readily understood by the manager of the worksite 12 such as "high risk", "low risk", "poor sound insulation effect", and "normal sound insulation effect". Thus, the manager of the worksite 12 can readily understand various assessment results (such as the noise-induced risk level, the sound insulation effect of the sound insulation device 100, etc.) from the assessment result information displayed by the terminal device 300 without having any specialized knowledge or without performing any special analysis or the like.

In the server 200, for example, the data storage unit 212 is implemented by a RAM 203 or an auxiliary storage device 204 (see FIG. 3B) included in the server 200. For example, the sound data acquisition unit 213, the sound pressure level calculator 214, the assessment unit 215, and the assessment result output unit 216 are implemented by executing a program by the CPU 201 (see FIG. 3B). For example, the communication unit 211 is implemented by the communication I/F 205 (see FIG. 3B) included in the server 200.

### <Function of terminal device 300>

As illustrated in FIG. 4, the terminal device 300 includes a communication unit 301, a setting unit 302, and a display controller 303.

The communication unit 301 transmits data to and receives data from an external device by communicating with the external device. For example, the communication unit 301 receives an assessment result transmitted from the server 200 through the communication network 18.

The setting unit 302 sets various parameter values (e.g., various thresholds) used in the assessment system 10 to each device disposed in the assessment system 10 in response to the setting operation by the manager of the worksite 12. For example, the setting unit 302 may set the sound insulation device 100 to be assessed, the assessment period, or the like to the server 200.

The display controller 303 controls the display by the display 207 included in the terminal device 300. For example, the display controller 303 displays various application screens for displaying various assessment results received by the communication unit 301 on the display 207.

In the terminal device 300, for example, the setting unit 302 and the display controller 303 are implemented by executing a program by the CPU 201 (see FIG. 3B). For example, the communication unit 301 is implemented by the communication I/F 205 (see FIG. 3B) disposed in the terminal device 300.

### (Example (first example) of the procedure for assessing the sound insulation effect by a server 200)

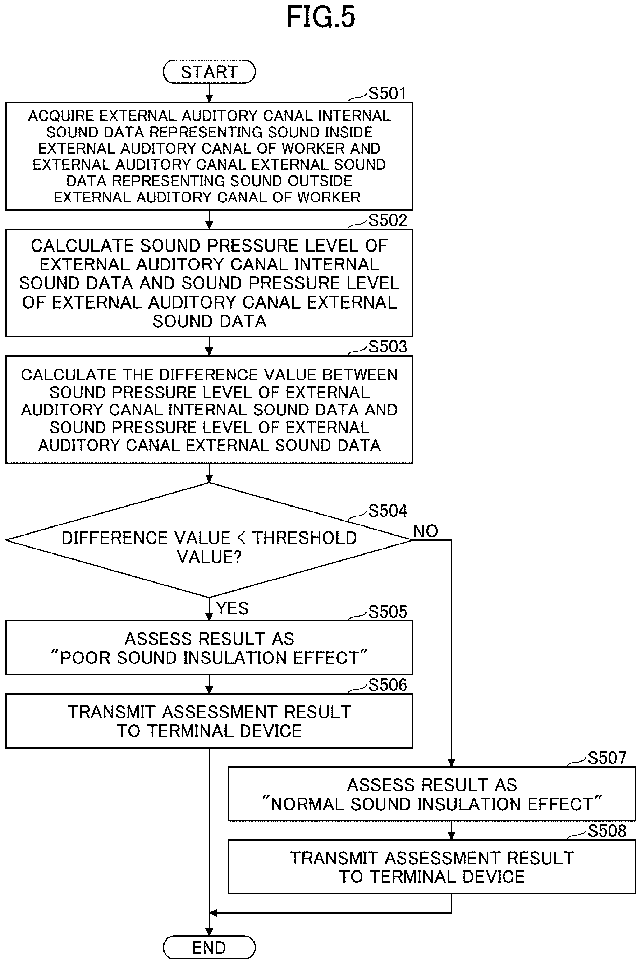

FIG. 5 is a flowchart illustrating an example (first example) of a procedure of a sound insulation assessment process by a server 200 according to the first embodiment of the present invention. Herein, an example of a procedure of a sound insulation effect assessment process performed by the server 200 when each of the fitting portions 100L and 100R disposed in the sound insulation device 100 to be assessed includes both the first microphone 104 and the second microphone 105 will be described.

First, in Step S501, the sound data acquisition unit 213 acquires, from the data storage unit 212, external auditory canal internal sound data representing sound inside the external auditory canal of the worker and external auditory canal external sound data representing sound outside the external auditory canal of the worker, where the external auditory canal internal sound data and the external auditory canal external sound data are acquired from the sound insulation device 100 to be assessed and stored in the data storage unit 212.

Next, in Step S502, the sound pressure level calculator 214 calculates the sound pressure level of the external auditory canal internal sound data acquired in Step S501 and the sound pressure level of the external auditory canal external sound data acquired in Step S501.

Next, in Step S503, the sound insulation effect assessment unit 215B calculates the difference value between the sound pressure level of the external auditory canal internal sound data calculated in Step S502 and the sound pressure level of the external auditory canal external sound data calculated in Step S502.

Then, in Step S504, the sound insulation effect assessment unit 215B determines whether or not the difference value of the sound pressure level calculated in Step S503 is less than a predetermined threshold value.

In Step S504, when the sound insulation effect assessment unit 215B determines that the difference value of the sound pressure level is less than the predetermined threshold value (Yes in Step S504), the sound insulation effect assessment unit 215B assesses the result as a "poor sound insulation effect" (Step S505). In Step S506, the assessment result output unit 216 transmits the assessment result information including the assessment result ("poor sound insulation effect") in Step S505 to the terminal device 300 through the communication unit 211 and the communication network 18. Thereafter, the server 200 ends the series of processes illustrated in FIG. 5.

In Step S504, conversely, when the sound insulation effect assessment unit 215B determines that the difference value of the sound pressure level is not less than the predetermined threshold value (No in Step S504), the sound insulation effect assessment unit 215B assesses the result as a "normal sound insulation effect" in Step S507. In step S508, the assessment result output unit 216 transmits the assessment result information including the assessment result ("normal sound insulation effect") in step S507 to the terminal device 300 through the communication unit 211 and the communication network 18. Thereafter, the server 200 ends the series of processes illustrated in FIG. 5.

### (Example (second example) of a procedure for assessing the sound insulation effect by a server 200)

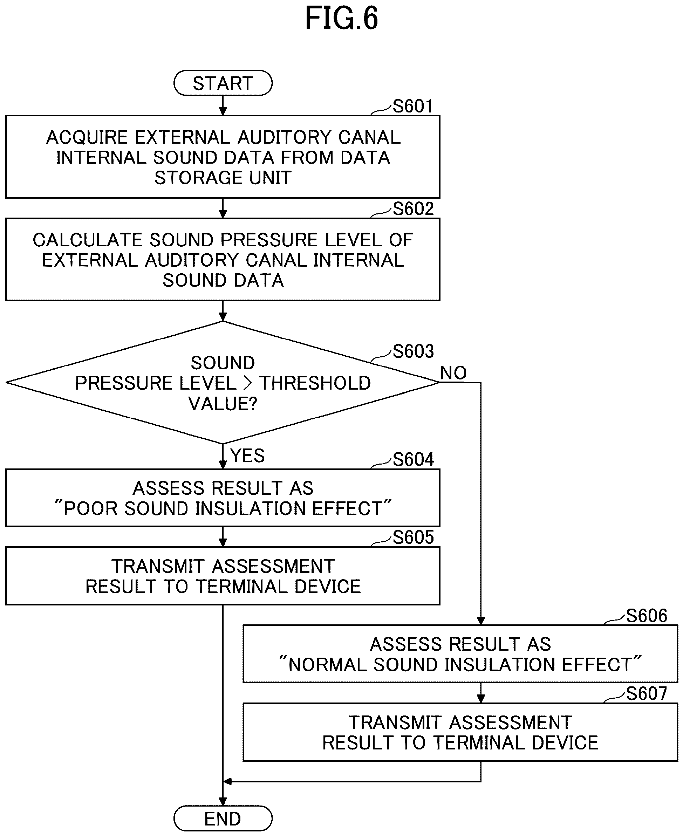

FIG. 6 is a flowchart illustrating an example (second example) of a procedure of a sound insulation assessment process by the server 200 according to the first embodiment of the present invention. Herein, an example of a procedure of a sound insulation effect assessment process performed by the server 200 when each of the fitting portions 100L and 100R disposed in the sound insulation device 100 to be assessed has only the first microphone 104 will be described.

First, in Step S601, the sound data acquisition unit 213 acquires the external auditory canal internal sound data of a worker representing the sound inside the external auditory canal obtained from the data storage unit 212 from the sound insulation device 100 to be assessed.

Next, in Step S602, the sound pressure level calculator 214 calculates the sound pressure level of the external auditory canal internal sound data acquired in Step S601.

Next, in Step S603, the sound insulation effect assessment unit 215B determines whether or not the sound pressure level of the external auditory canal internal sound data calculated in Step S603 is greater than the predetermined threshold value.

In Step S603, when the sound insulation effect assessment unit 215B determines that the sound pressure level of the external auditory canal internal sound data is greater than a predetermined threshold value (Yes in Step S603), the sound insulation effect assessment unit 215B assesses the result as a "poor sound insulation effect" (Step S604). In Step S605, the assessment result output unit 216 transmits the assessment result information including the assessment result ("poor sound insulation effect") in Step S604 to the terminal device 300, through the communication unit 211 and the communication network 18. Thereafter, the server 200 ends the series of processes illustrated in FIG. 6.

In Step S603, conversely, when the sound insulation effect assessment unit 215B determines that the sound pressure level of the external auditory canal internal sound data is not greater than the predetermined threshold value (No in Step S603), the sound insulation effect assessment unit 215B assesses the result as a "normal sound insulation effect" in Step S606. In Step S607, the assessment result output unit 216 transmits the assessment result information including the assessment result ("normal sound insulation effect") in Step S606 to the terminal device 300 through the communication unit 211 and the communication network 18. The server 200 then ends the series of processes illustrated in FIG. 6.

The terminal device 300 outputs the assessment result information transmitted from the server 200 through a series of processes illustrated in FIGS. 5 and 6 (e.g., the assessment result information is displayed by the display 207) so that the manager can understand a fitting condition with respect to each sound insulation device 100. The manager may then, for example, urge a user of the sound insulation device 100 assessed as "poor sound insulation effect" to properly fit the sound insulation device 100.

FIGS. 5 and 6 each illustrate a procedure of an assessment process of a sound insulation effect with respect to one of the fitting portions 100L and 100R disposed in the sound insulation device 100 to be assessed. That is, the server 200 can assess a sound insulation effect of the fitting portion 100L and 100R by performing a sound insulation effect assessment process exemplified in FIG. 5 or FIG. 6 for each of the fitting portions 100L and 100R.

Further, in the sound insulation effect assessment process illustrated in FIGS. 5 and 6, the assessment result output unit 216 is configured to transmit the assessment result information to the terminal device 300; however, the configuration is not limited to this example. For example, the assessment result output unit 216 may transmit the assessment result information to the sound insulation device 100 to notify a user of the sound insulation device 100 of the assessment result of the sound insulation effect. The server 200 may store the assessment result information in the data storage unit 212 such that the assessment result information can be collectively output at any time.

### (Example of display screen)

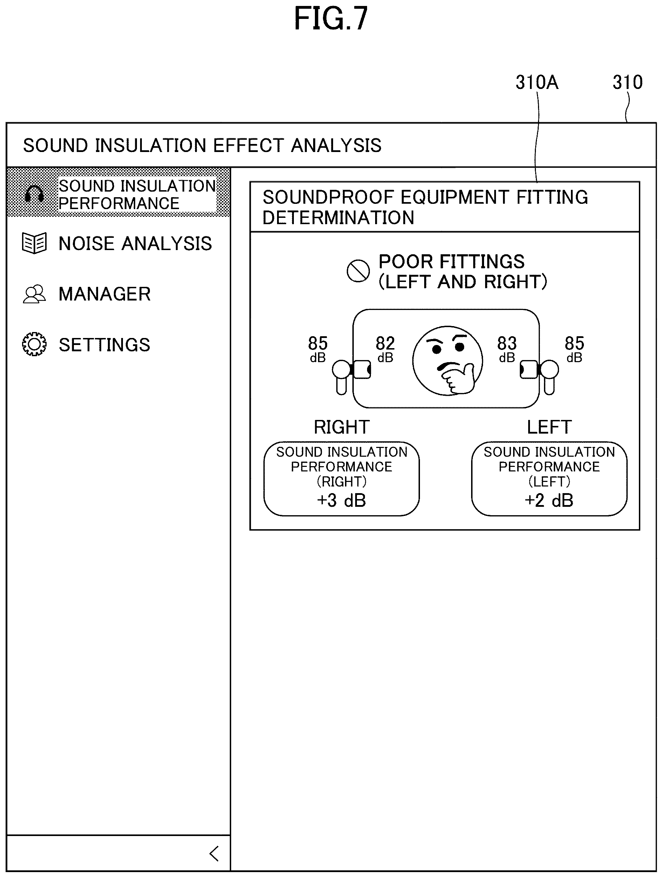

FIG. 7 is a diagram illustrating an example (first example) of a display screen displayed on the terminal device 300 according to the first embodiment of the present invention. The display screen 310 illustrated in FIG. 7 is an example of a display screen displayed on the display 207 of the terminal device 300. The display screen 310 is an application screen of a noise analysis application that represents an assessment result of the sound insulation effect with respect to one sound insulation device 100.

In the display screen 310 illustrated in FIG. 7, a sound pressure level of the external auditory canal external sound data acquired by the second microphone 105 and a sound pressure level of the external auditory canal internal sound data acquired by the first microphone 104 are displayed in the display field 310A, for each of the fitting portions 100L and 100R included in the sound insulation device 100.

In the display screen 310 illustrated in FIG. 7, the difference value between the sound pressure level of the external auditory canal external sound data and the sound pressure level of the external auditory canal internal sound data is displayed as a sound insulation performance in the display field 310A, for each of the fitting portions 100L and 100R disposed in the sound insulation device 100.

Further, in the display screen 310 illustrated in FIG. 7, an assessment result of the sound insulation effect ("poor fittings") is displayed in the display field 310A, for each of the fitting portions 100L and 100R included in the sound insulation device 100, based on the difference value between the sound pressure level of the external auditory canal external sound data and the sound pressure level of the external auditory canal internal sound data.

The display contents are displayed in the display field 310A of the display screen 310 by the control of the display controller 303 included in the terminal device 300 based on the assessment result information transmitted from the server 200.

### (Example of procedure for risk assessment process by server 200)

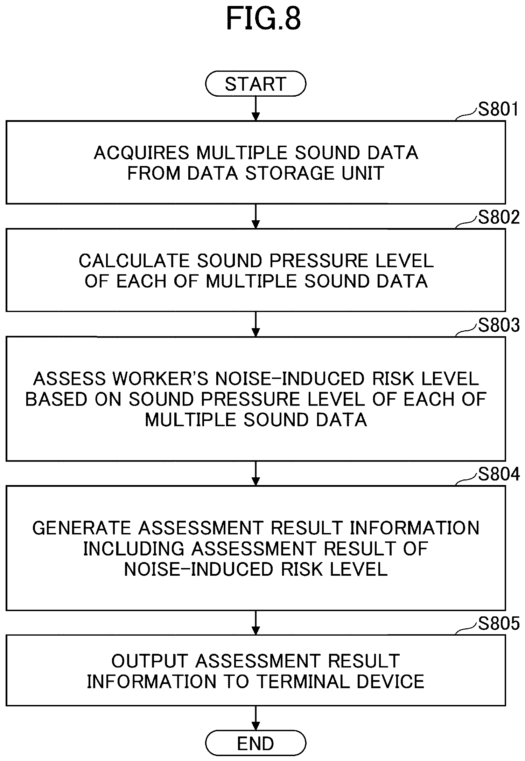

FIG. 8 is a flowchart illustrating an example of a procedure of a risk assessment process by a server 200 according to the first embodiment of the present invention.

First, in Step S801, the sound data acquisition unit 213 acquires sound data (external auditory canal internal sound data and external auditory canal external sound data) from the data storage unit 212 within the assessment period (e.g., 1 hour or 1 day) of the sound insulation device 100 (e.g., any one device, any two or more of devices, all the devices, etc.) to be assessed.

Next, in Step S802, the sound pressure level calculator 214 calculates each of the sound pressure levels of the plurality of sound data sets acquired in Step S801.

Next, in Step S803, the risk assessment unit 215A assesses the noise-induced risk level with respect to the sound insulation device 100 (a worker) to be assessed, based on the sound pressure level of each of the plurality of sound data sets calculated in Step S802 and time information associated with a corresponding one of the sound pressure levels.

For example, the risk assessment unit 215A assesses, based on the sound pressure level of each of the plurality of sound data sets calculated in step S802 and the time information associated with a corresponding one of the plurality of sound data sets calculated in step S802, the noise-induced risk level every predetermined time unit, with respect to the sound insulation device 100 (the worker) to be assessed.

Also, for example, the risk assessment unit 215A assesses the noise-induced risk level according to the noise-exposed cumulative time with respect to the sound insulation device 100 (the worker) to be assessed, based on the sound pressure level of each of the plurality of sound data sets calculated in step S802 and the time information associated with a corresponding one of the plurality of sound data sets calculated in step S802.

In Step S804, the risk assessment unit 215A generates assessment result information including the assessment result of the noise-induced risk level with respect to the worker obtained in Step S803 and information (sound pressure level, device ID, position information, time information, etc.) associated with the assessment result.

In step S805, the assessment result output unit 216 outputs the assessment result information generated in step S804 to the terminal device 300 through the communication unit 211 and the communication network 18. Thereafter, the server 200 ends the series of processes illustrated in FIG. 8.

In the terminal device 300, when the communication unit 301 receives the assessment result information transmitted from the server 200 in step S804, the display controller 303 displays the assessment result information on the display 207 (see FIG. 3B) included in the terminal device 300 through a predetermined application screen. This allows the manager to understand the risk level caused by noise for each worker at the worksite 12.

### (Example of assessment result information output by the assessment result output unit 216 (first example))

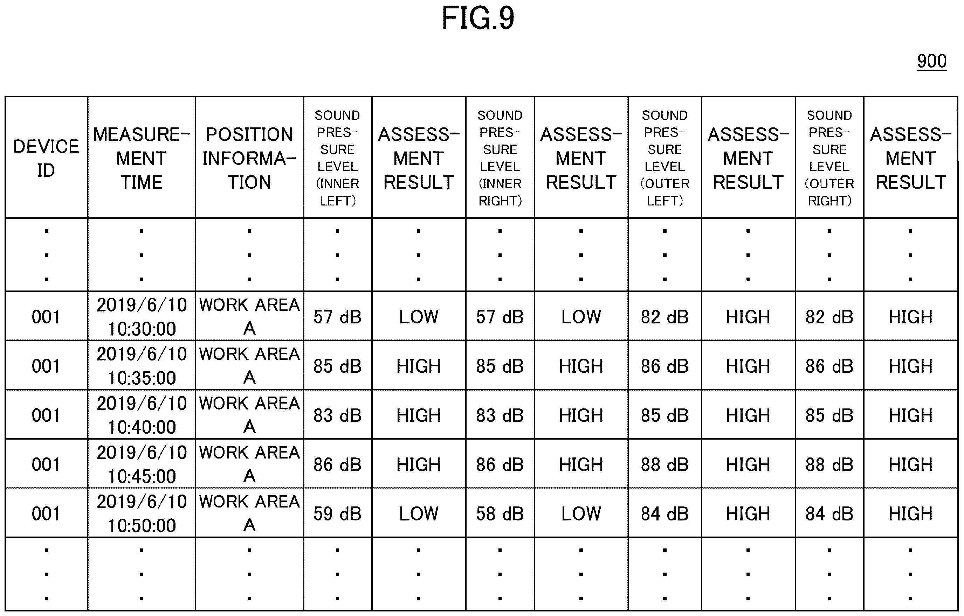

FIG. 9 is a diagram illustrating an example (first example) of assessment result information output by the assessment result output unit 216 according to the first embodiment of the present invention.

The assessment result information 900 illustrated in FIG. 9 represents the results of the noise-induced risk level assessment at every predetermined time unit with respect to a single sound insulation device 100 (i.e., a single worker). As illustrated in FIG. 9, the assessment result information 900 includes the device ID, the position information, the sound pressure levels of the left and right external auditory canal internal sound data and the sound pressure levels of the left and right external auditory canal external sound data, and the assessment result of the risk level of each of the sound pressure levels of each sound data at every predetermined time unit (in the example illustrated in FIG. 9, every 5 minutes).

For example, in the assessment result information 900 illustrated in FIG. 9, when the sound pressure level of each sound data is greater than a predetermined threshold value, "high" is set as the assessment result of the risk level, and when the sound pressure is lower than the predetermined threshold value, "low" is set as the assessment result of the risk level.

It should be noted that, in the assessment result information 900 illustrated in FIG. 9, the assessment result for the sound pressure level outside the external auditory canal represents the noise-induced risk level itself generated at the worksite. Meanwhile, in the assessment result information 900 illustrated in FIG. 9, the assessment result for the sound pressure level inside the external auditory canal represents the noise-induced risk level of the noise actually entering the external auditory canal of a worker.

For example, the assessment result information 900 is transmitted from the server 200 to the terminal device 300. The terminal device 300 displays the assessment result information 900 on the display 207. This enables the manager of the worksite 12 to understand the noise-induced risk level with respect to a worker on a per predetermined time unit basis.

Specifically, since the assessment result information 900 includes position information and time information, the manager of the worksite 12 can readily understand which work area is at high noise-induced risk on a per predetermined time unit basis.

Furthermore, since the assessment result information 900 uses a display format by which the risk level is readily understood by the manager, such as "high" or "low" as the assessment result of the risk level, the manager of the worksite 12 can readily understand on a per predetermined time unit basis which work area the noise-induced risk level is high based on the assessment result information 900.

In the example illustrated in FIG. 9, the assessment result information 900 includes the assessment result information with respect to one sound insulation device 100 since one sound insulation device 100 is subject to assessment (an assessment target). When the plurality of sound insulation devices 100 is the subject of the assessment, the assessment result information 900 includes the assessment result information regarding the plurality of sound insulation devices 100.

### (Example of assessment result information output by the assessment result output unit 216 (second example))

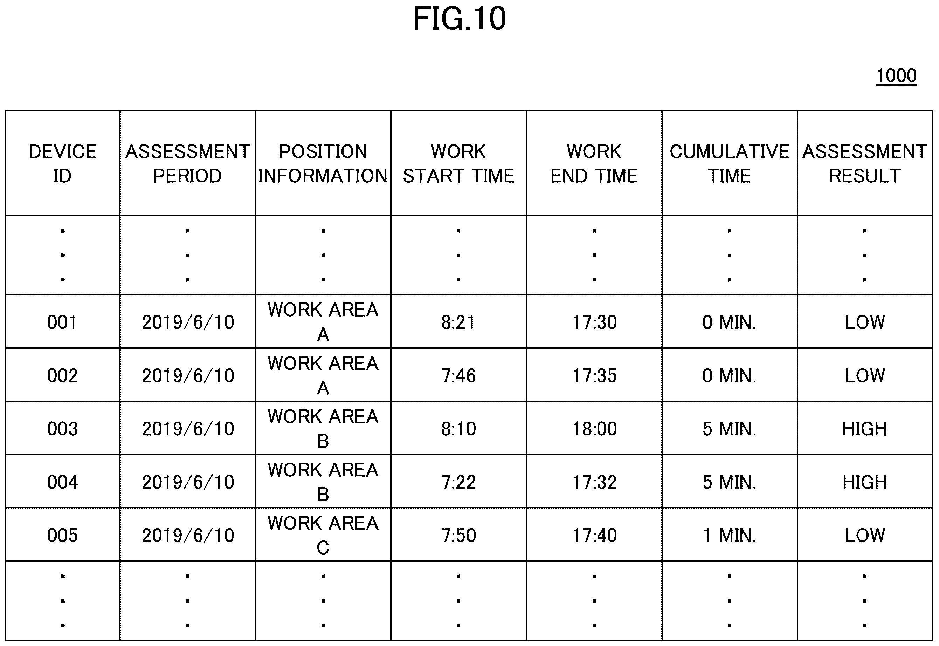

FIG. 10 is a diagram illustrating an example (a second example) of assessment result information output by the assessment result output unit 216 according to the first embodiment of the present invention.

The assessment result information 1000 illustrated in FIG. 10 represents assessment results of the risk level due to the noise exposure cumulative time with respect to each of the plurality of sound insulation devices 100 (i.e., the plurality of workers). As illustrated in FIG. 10, the assessment result information 1000 includes the assessment results of the risk level due to the noise exposure cumulative time with respect to each of the plurality of sound insulation devices 100 and associated information of the assessment results (device ID, assessment period, position information, work start time, work end time, and cumulative time).

In the example illustrated in FIG. 10, the assessment result information 1000 illustrates the assessment results in terms of the noise exposure cumulative time with respect to each of the plurality of sound insulation devices 100 for a certain day ("2019/6/10") as the assessment period.

For example, in the assessment result information 1000 illustrated in FIG. 10, the noise exposure cumulative time of the device IDs "003" and "004" (cumulative time during which the sound pressure level is greater than a predetermined threshold) is set to "5 minutes". This "5 minutes" is a time longer than a predetermined allowable time. Accordingly, in the assessment result information 1000 illustrated in FIG. 10, the assessment result of the risk level due to the noise exposure cumulative time is set to "high" with respect to the device IDs "003" and "004".

For example, the assessment result information 1000 is transmitted from the server 200 to the terminal device 300. The terminal device 300 displays the assessment result information 1000 on the display 207. This enables the manager of the worksite 12 to readily understand the risk level due to the noise exposure cumulative time with respect to each of the multiple workers.

Specifically, since the assessment result information 1000 includes position information, the manager of the worksite 12 can readily understand which work area is at high risk due to noise exposure cumulative time.

Furthermore, since the assessment result information 1000 uses a display format by which the risk level is readily understood by the manager, such as "high" or "low" as the assessment result of the risk level, the manager of the worksite 12 can readily understand which work area is at a highly risk on a per predetermined time unit basis, based on the assessment result information 1000.

### (Example of display screen (example 2))

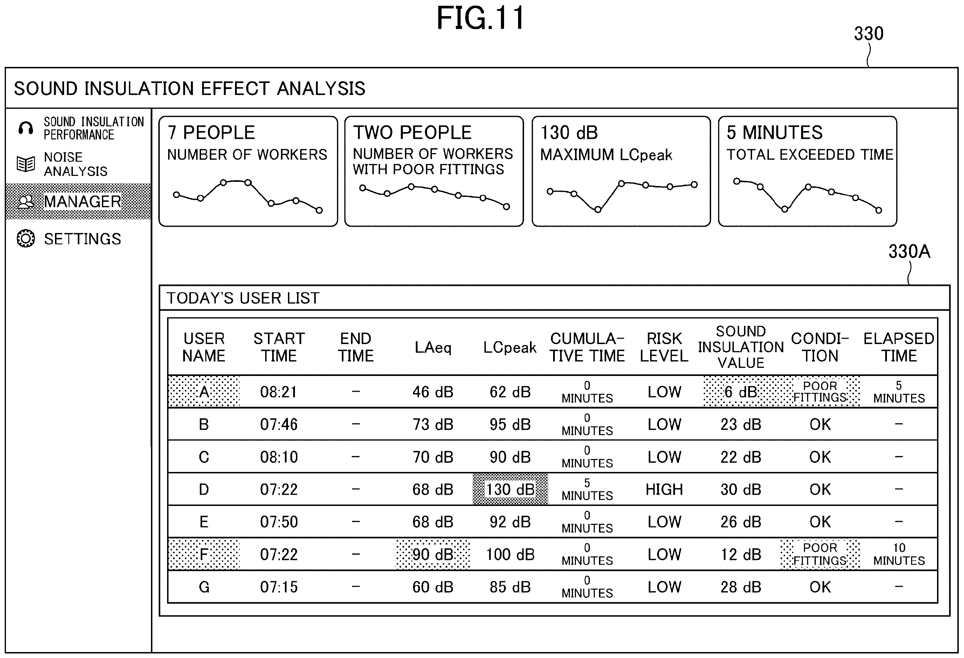

FIG. 11 is a diagram illustrating an example (a second example) of a display screen displayed on the terminal device 300 according to the first embodiment of the present invention. A display screen 330 illustrated in FIG. 11 is another example of the display screen displayed on the display 207 of the terminal device 300. The display screen 330 displays a list of various assessment results of each of the plurality of workers assessed.

For example, in the example illustrated in FIG. 11, for each of the seven workers to be assessed (user IDs "A" to "G"), the "start time", "end time", "LAeq", "LCpeak", "cumulative time", "risk level", "sound insulation value", "condition", and "elapsed time" are displayed on the list display field 330A of the display screen 330.

The "start time" indicates the start time of work started by a worker as specified by the risk assessment unit 215A of the server 200. The "end time" indicates the end time of the work ended by the worker as specified by the risk assessment unit 215A of the server 200. In the example illustrated in FIG. 11, a "-" is set for the "end time" of each worker, indicating that each worker is currently working. "LAeq" indicates the equivalent noise level (when the noise level varies irregularly and significantly with time (unsteady sound, fluctuating noise), the time mean value is calculated focusing on the energy of the noise level that varies within a certain period of time), and "LCpeak" indicates the C-weighted peak sound pressure level (the value obtained by converting the maximum value of the absolute value of the instantaneous sound pressure (AC output waveform) during the measurement time to the dB value and weighted by the C-weighted value). "Cumulative time" indicates the cumulative time during which the sound pressure level is greater than a predetermined threshold value calculated by the risk assessment unit 215A of the server 200. The "risk level" indicates the assessment result of the risk level ("low" or "high") based on the cumulative time set as "cumulative time" as determined by the risk assessment unit 215A of the server 200. The "sound insulation value" represents the difference value between the sound pressure level of the external auditory canal internal sound data and the sound pressure level of the external auditory canal external sound data calculated by the sound insulation effect assessment unit 215B of the server 200. The "condition" indicates the assessment result of the sound insulation effect of the sound insulation device 100 ("poor fittings" or "OK") determined by the sound insulation effect assessment unit 215B of the server 200. The "elapsed time" indicates the elapsed time since the sound insulation effect of the sound insulation device 100 has been determined to be "poor fittings" by the sound insulation effect assessment unit 215B of the server 200.

In addition, the number of workers subject to assessment ("7 people"), the number of workers determined to be "poor fittings" ("two people"), the maximum value of the LCpeak ("130 dB"), and the total value of the excess time ("5 minutes") are displayed outside the list display field 330A of the display screen 330.

Each of the information displayed on the display screen 330 is derived from the plurality of sound data sets within the assessment period by the assessment unit 215 of the server 200, and is output to the terminal device 300 as assessment result information by the assessment result output unit 216 of the server 200. The manager of the worksite 12 can readily understand from this display screen 330, which is displayed on the display 207 of the terminal device 300, the assessment results including time factors of each worker working at the worksite 12, such as the noise-induced risk level, the sound insulation effect of the sound insulation device 100, and the like. In particular, in the display screen 330, each assessment result is illustrated in a display format that is readily understood by the manager of the worksite 12. Therefore, the manager of the worksite 12 can readily understand various assessment results (the noise-induced risk level, the sound insulation effect of the sound insulation device 100, etc.) from the display screen 330 without having specialized knowledge or without performing special analysis.

As described above, the assessment system 10 according to the first embodiment includes the sound data acquisition unit 213 configured to acquire sound data collected by the sound insulation device 100 fitted into the worker's (wearer's) ears, and the assessment unit 215 for performing assessment of the noise being exposed by the worker based on sound data acquired by the sound data acquisition unit 213 and time information associated with sound data. Thus, in the assessment system 10 according to the first embodiment, the assessment including the time factors with respect to the noise to which the wearer is actually exposed can be made, based on the noise level to which the wearer is actually exposed.

In the assessment system 10 according to the first embodiment, at least a part of the functions of the server 200 (see FIG. 4) may be disposed in the terminal device 300, and the functions of the server 200 may be implemented by the server 200 and the terminal device 300. For example, the data storage unit 212 may be disposed in the server 200, and the sound data acquisition unit 213, the sound pressure level calculator 214, and the assessment unit 215 may be disposed in the terminal device 300.

For example, the terminal device 300 may store a plurality of sound data sets collected from a plurality of sound insulation data in place of the server 200 and perform various assessments (e.g., assessment of the risk level by the noise pressure level, assessment of the sound insulation effect of the sound insulation device 100, detection of abnormal sound at the worksite 12, etc.) relating to the noise to which the worker is exposed, based on the plurality of sound data sets.

In addition, the server 200 can increase the real-time properties of the assessment process by performing a process of assessing the sound insulation effect in shorter time units (e.g., in seconds or minutes). This enables, for example, the manager of the worksite 12 to immediately instruct a worker, who uses the sound insulation device 100 that is determined to have a poor sound insulation effect based on the assessment result of the sound insulation effect displayed in real time, to correctly wear the sound insulation device 100.

In addition, the server 200 can increase the real-time properties of the noise-induced risk assessment process by performing a process of assessing the noise-induced risk assessment in shorter time units (e.g., in seconds or minutes). This may, for example, enable the manager of the worksite 12 to immediately instruct workers wearing the sound insulation device 100 who are determined to be at high risk to take countermeasures (e.g., to stop the work, to wait until the risk level is reduced, to change the work place to a place with a lower risk level, to rest, etc.), based on the results of an assessment of the noise-induced risk level displayed in real time.

### [Second Embodiment]

### (System configuration of assessment system 10A)

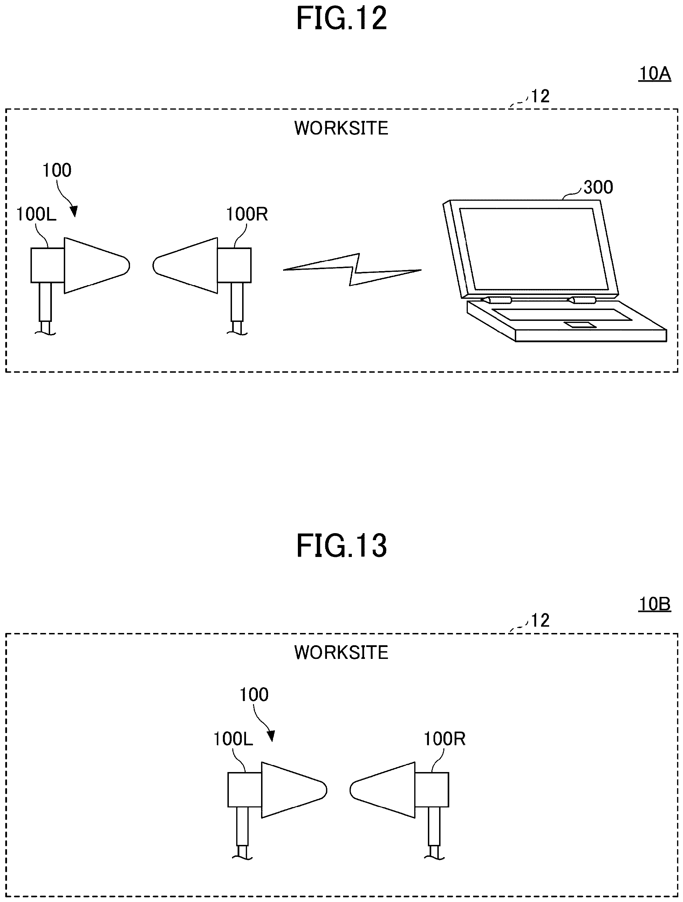

FIG. 12 is a diagram illustrating a system configuration of an assessment system 10A according to a second embodiment of the present invention.

The assessment system 10A illustrated in FIG. 12 differs from the assessment system 10 according to the first embodiment in that there is no server 200, and the sound insulation device 100 and the terminal device 300 are disposed such that the sound insulation device 100 and the terminal device 300 are capable of performing wireless communication or wired communication with each other at the worksite 12.

For example, in the assessment system 10A according to the second embodiment, the functions of the server 200 described in the first embodiment (the function of collecting the sound data from the sound insulation device 100, the function of storing the sound data, and the various assessment functions) are disposed in the terminal device 300. Therefore, the functions of the server 200 described in the first embodiment can be implemented by the terminal device 300 without the server 200.

For example, in the assessment system 10A according to the second embodiment, when the sound insulation device 100 is provided with the sound insulation effect assessment function of the server 200 described in the first embodiment, the sound insulation device 100 is capable of assessing the sound insulation effect of the sound insulation device 100 itself.

In this case, for example, the sound insulation device 100 may inform the worker of the assessment result of the x by at least one of the speaker 108, the LED 109, and the vibration generator 110.

Alternatively, the sound insulation device 100 can directly transmit the assessment result of the sound insulation effect to the terminal device 300 through wireless communication with the terminal device 300 by the communication unit 127, thereby notifying the manager of the worksite 12 of the assessment result.

Further, the sound insulation device 100 can increase the real-time properties of the sound insulation process by performing a process of assessing the sound insulation effect at every shorter time unit (e.g., in seconds or minutes). This enables, for example, the manager of the worksite 12 to instruct workers with poor sound insulation to immediately fit the sound insulation device 100 correctly.

### [Third Embodiment]

### (System configuration of assessment system 10B)

FIG. 13 is a diagram illustrating a system configuration of an assessment system 10B according to a third embodiment of the present invention. The assessment system 10B illustrated in FIG. 13 differs from the assessment system 10A according to the second embodiment in that only the sound insulation device 100 is provided at the worksite 12.

For example, in the assessment system 10B according to the third embodiment, the sound insulation device 100 can assess sound insulation effect of the sound insulation device 100 itself by providing the sound insulation device 100 with a function for calculating the sound pressure level of the server 200 or a function for assessing the sound insulation effect described in the first embodiment.

In this case, the sound insulation device 100 may, for example, inform the worker of the assessment result of the sound insulation effect by at least one of the speaker 108, the LED 109, and the vibration generator 110.

Further, the sound insulation device 100 executes an assessment process of assessing the sound insulation effect in shorter time units (e.g., in seconds and minutes) to increase the real-time properties of the assessment process of the sound insulation effect every shorter time unit (e.g., in seconds or minutes). This enables the sound insulation device 100 to immediately urge a worker having a poor sound insulation condition to correctly wear the sound insulation device 100.

Although the first to third embodiments of the present invention have been described in detail above, the present invention is not limited to these embodiments, and various modifications or alternations may be made within the scope of the subject matter of the present invention as claimed.

For example, in each of the embodiments described above, the sound insulation device 100 is configured to acquire the external auditory canal internal sound data and the external auditory canal external sound data by the first and second microphones 104 and 105. However, the embodiments are not limited to this example. For example, the sound insulation device 100 may measure the sound pressure level of the external auditory canal internal sound data and the sound pressure level of the external auditory canal external sound data by a sound pressure level measuring device disposed in the sound insulation device 100. The sound insulation device 100 may store the sound pressure level data representing the measured sound pressure levels and may transmit the sound pressure level data to the server 200 at any time. Further, the assessment unit 215 of the server 200 may perform various assessments, based on the sound pressure level data collected from the sound insulation device 100.

The sound insulation device 100 may further include a sound pressure level calculator configured to calculate a sound pressure level of sound data acquired by the first microphone 104 and a sound pressure level of sound data acquired by the second microphone 105. In this case, instead of storing each sound data, the data storage unit 120 may store sound pressure level data representing the sound pressure level of each sound data. The communication unit 127 may transmit the sound pressure level data stored in the data storage unit 120 to the server 200, instead of transmitting each sound data to the server 200 at any time. Further, the assessment unit 215 of the server 200 may perform various assessments, based on the sound pressure level data collected from the sound insulation device 100.

This international application claims priority under Japanese Patent Application No. 2019-141547, filed with the Japanese Patent Office on July 31, 2019, and the entire contents of Japanese Patent Application No. 2019-141547 are incorporated herein by reference.

### [Description of Reference Codes]

- 10,10A,10B: assessment system
- 12: work site
- 18: communication network
- 100: sound insulation device
- 100L: fitting portion
- 100R: fitting portion
- 101: housing
- 102: tube
- 103: sound insulation member
- 104: 1st microphone
- 105: 2nd microphone
- 120: data storage unit
- 127: communication unit
- 200: server
- 211: communication unit
- 212: data storage unit
- 213: sound data acquiring unit
- 214: sound pressure level calculator
- 215: assessment unit
- 215A: risk assessment unit
- 215B: sound insulation effect assessment unit
- 215C: abnormal sound detector
- 215D: utterance signal extraction unit
- 216: assessment result output unit
- 300: terminal device
- 301: communication unit
- 302: setting unit
- 303: display controller

## Claims

1. An assessment system comprising:
a sound data acquiring unit configured to acquire sound data collected by a sound insulation device, the sound insulation device being fitted into a wearer's ears; and
an assessment unit configured to assess noise to which the wearer is exposed, based on the sound data acquired by the sound data acquiring unit and time information associated with the sound data.

2. The assessment system according to claim 1, further comprising:
an assessment result output unit configured to output assessment result information representing an assessment result based on the sound data and the time information by the assessment unit.

3. The assessment system according to claim 1 or 2,
wherein the sound data acquiring unit acquires sound data collected by the sound insulation device, the sound data including one of or both of external auditory canal internal sound data and external auditory canal external sound data, the external auditory canal internal sound data representing sound inside the external auditory canal of the wearer, and the external auditory canal external sound data representing sound outside the external auditory canal of the wearer, and
wherein the assessment unit assesses noise to which the wearer is exposed, based on: the one of or both of external auditory canal internal sound data and external auditory canal external sound data; and time information associated with a corresponding one of or both of the external auditory canal internal sound data and the external auditory canal external sound data.

4. The assessment system according to claim 1 or 2,
wherein the assessment unit assesses noise to which the wearer is exposed, based on the sound pressure level of the sound data acquired by the sound data acquiring unit and the time information associated with the sound data.

5. The assessment system as claimed in claim 3,
wherein the assessment unit includes a sound insulation effect assessment unit configured to assess a sound insulation effect of the sound insulation device, based on a difference value between a sound pressure level of the external auditory canal internal sound data acquired by the sound data acquiring unit and a sound pressure level of the external auditory canal external sound data acquired by the sound data acquiring unit.

6. The assessment system according to claim 1 or 2,
wherein the assessment unit includes a risk assessment unit configured to assess a noise-induced risk at every predetermined time unit, based on the sound data acquired by the sound data acquiring unit and the time information associated with the sound data.

7. The assessment system according to claim 1 or 2,
wherein the assessment unit includes a risk assessment unit configured to assess a risk due to noise exposure cumulative time, based on the sound data acquired by the sound data acquiring unit and time information associated with the sound data.

8. The assessment system according to claim 1 or 2,
wherein the assessment unit includes an abnormal sound detector configured to detect abnormal sound at a noise generating location, based on the sound data acquired by the sound data acquiring unit.

9. The assessment system according to claim 1 or 2,
wherein the assessment unit includes an utterance signal extraction unit configured to extract utterance signals representing speech uttered by the wearer, from the sound data acquired by said sound data acquiring unit.

10. An assessment device comprising:
a sound data acquiring unit configured to acquire sound data collected by a sound insulation device, the sound insulation device being fitted into a wearer's ears; and
an assessment unit configured to assess noise to which the wearer is exposed, based on the sound data acquired by the sound data acquiring unit and time information associated with the sound data.

11. An assessment method comprising:
acquiring sound data collected by a sound insulation device, the sound insulation device being fitted into a wearer's ears; and
assessing noise to which the wearer is exposed, based on the sound data acquired in the acquiring and time information associated with the sound data.

12. A program for causing a computer to function as:
a sound data acquiring unit configured to acquire sound data collected by a sound insulation device, the sound insulation device being fitted into a wearer's ears; and
an assessment unit configured to assess noise to which the wearer is exposed, based on the sound data acquired by the sound data acquiring unit and time information associated with the sound data.

13. A sound insulation device to be fitted into a wearer's ears, the sound insulation device comprising:
a sound data acquiring unit configured to acquire sound data collected by the sound insulation device; and
an assessment unit configured to assess noise to which the wearer is exposed, based on the sound data acquired by the sound data acquiring unit and time information associated with the sound data.
